# EUROPEAN PATENT APPLICATION

(11) **EP 3 482 672 A1**
(43) Date of publication of application: **15.05.2019**
(21) Application number: 17827230.8
(22) Date of filing: 11.05.2017
(51) Int. Cl.: A61B 1/045, A61B 1/00, A61B 1/04, A61B 1/07, G02B 23/24

(54) **ENDOSCOPE DEVICE, AND IMAGE SYNTHESIS METHOD FOR ENDOSCOPE DEVICE**

(30) Priority: 12.07.2016 JP 2016138036
(71) Applicant: Sony Corporation, Tokyo 108-0075 (JP)
(72) Inventor: YAMAGUCHI, Kenta, Tokyo 108-0075 (JP); MIYAI, Takeshi, Tokyo 108-0075 (JP); FUKAZAWA, Kentaro, Tokyo 108-0075 (JP); KASHIMA, Koji, Tokyo 108-0075 (JP)
(74) Representative: D Young & Co LLP
(86) International application number: PCT/JP2017/017768
(87) International publication number: WO 2018/012096

(57) **Abstract**

[Object] To optimally adjust the specular refection component without lowered luminance.

[Solution] An endoscope device according to the present disclosure includes: a reflection-presence image acquisition unit that acquires a reflection-presence image containing a specular reflection component from a subject; a reflection-absence image acquisition unit that acquires a reflection-absence image not containing the specular reflection component from the subject; and a synthesis processing unit that performs synthesis of the reflection-presence image and the reflection-absence image. This configuration makes it possible to optimally adjust the specular refection component without lowered luminance, and makes it possible to optimally perform observation of the subject.

## Description

### Technical Field

The present disclosure relates to an endoscope device, and an image synthesis method for the endoscope device.

### Background Art

As a technique for improving low visibility due to a magnitude of brightness difference in an endoscope observation, it is described in, for example, Patent Literature 1 below that the amount of irradiation light and a light distribution pattern according to distance information are changed, and variation in light distribution is adjusted by gain correction to present a video of correct exposure.

Also, in Patent Literature 2 below, the following configuration is described: as an imaging technique for removing a specular reflection image, when first illumination light that is illumination light coming from a first light source is polarized at a first polarizing plate to fall on an object, and imaging light from the object passes through the first polarizing plate and a polarizing plate of which the polarization direction is orthogonal to the first polarizing plate, surface reflected light is set to be cut off.

### Citation List

### Patent Literature

Patent Literature 1: JP 2015-8785A
Patent Literature 2: JP 2014-18439A

### Disclosure of Invention

### Technical Problem

However, in a method described in Patent Literature 1, there is a problem that even when correct illuminance to a subject can be implemented, a video suitable for observation cannot be presented due to a specular reflection component from the subject.

Also, in the technique described in Patent Literature 2, a reflection-absence image having no surface reflected wave (specular reflection component) to be acquired is difficult to be used for normal observation purposes because it is different in texture and impression of the subject from a normal image having the specular reflection component.

Further, in the technique described in Patent Literature 2, as a configuration for removal of the specular reflection imaging light, since a polarizer is intervened for each of irradiated light and reflected light, the amount of light entering an imaging section will be less than 1/4 of the amount of irradiation light, as compared to a case not through a polarizing plate. For this reason, there is a problem that S/N of a video is significantly impaired.

Therefore, it has been demanded to optimally adjust the specular refection component without lowered luminance.

### Solution to Problem

According to the present disclosure, there is provided an endoscope device including: a reflection-presence image acquisition unit that acquires a reflection-presence image containing a specular reflection component from a subject; a reflection-absence image acquisition unit that acquires a reflection-absence image not containing the specular reflection component from the subject; and a synthesis processing unit that performs synthesis of the reflection-presence image and the reflection-absence image.

In addition, according to the present disclosure, there is provided an image synthesis method for an endoscope device, including: acquiring a reflection-presence image containing a specular reflection component from a subject; acquiring a reflection-absence image not containing the specular reflection component from the subject; and performing synthesis of the reflection-presence image and the reflection-absence image.

### Advantageous Effects of Invention

As described above, according to the present disclosure, it becomes possible to optimally adjust the specular refection component without lowered luminance.

Note that the effects described above are not necessarily limitative. With or in the place of the above effects, there may be achieved any one of the effects described in this specification or other effects that may be grasped from this specification.

### Brief Description of Drawings

FIG. 1 is a schematic diagram for illustrating a schematic configuration of a system in accordance with one embodiment of the present disclosure.
FIG. 2 is an explanatory diagram showing one example of a hardware configuration of a CCU in FIG. 1.
FIG. 3A is a schematic diagram showing a state in which a subject is lighted with uniform illuminance by the sun's rays such as under a natural environment.
FIG. 3B is a schematic diagram showing a state in which a subject is lighted by a single light source in a blocked state of external light as in an endoscopic observation.
FIG. 4 is a schematic diagram showing a structure of an endoscopic device.
FIG. 5A is a schematic diagram showing a relationship between irradiated light and incident light during observation in normal photographing.
FIG. 5B is a schematic diagram showing a relationship between irradiated light and incident light during observation in normal photographing.
FIG. 5C is a schematic diagram showing a relationship between irradiated light and incident light during observation in normal photographing.
FIG. 6 is a schematic diagram showing a methodology using a spatial division system.
FIG. 7 is a schematic diagram showing an example in a spatial arrangement of a polarization pixel and a normal pixel.
FIG. 8 is a schematic diagram showing a methodology for synthesizing a reflection-presence image and a reflection-absence image acquired by the spatial division system.
FIG. 9A is a schematic diagram showing a methodology using a time division system.
FIG. 9B is a schematic diagram showing a methodology using a time division system.
FIG. 10 is a schematic diagram showing an example in which for acquisition of the reflection-presence image and the reflection-absence image in the time division system, a switch between a first polarizer and a second polarizer is performed together with a switch between color filters (R, G, B).
FIG. 11A is a schematic diagram showing a methodology using a light beam split system.
FIG. 11B is a schematic diagram showing a methodology using a light beam split system.
FIG. 12 is a schematic diagram showing an example in which a direction of polarization is changed through the use of a phase difference plate.
FIG. 13 is a schematic diagram for illustrating a synthesized image producing unit that produces a synthesized image from the reflection-absence image and the reflection-presence image.
FIG. 14 is a schematic diagram for illustrating a synthesized image producing unit that produces a synthesized image from the reflection-absence image and the reflection-presence image.
FIG. 15A is a characteristic graph showing a use rate coefficients α1 in synthesis.
FIG. 15B is a characteristic graph showing a use rate coefficients α2 in synthesis.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that the description will be made in the following order.
1. Entire Configuration of System
2. Configuration of Endoscope in accordance with Present Embodiment
3. Example of Spatial Division System
4. Example of Time Division System
5. Example of Light Beam Split System
6. Synthesis of Reflection-Absence Image and Reflection-Presence Image

### [1. Entire Configuration of System]

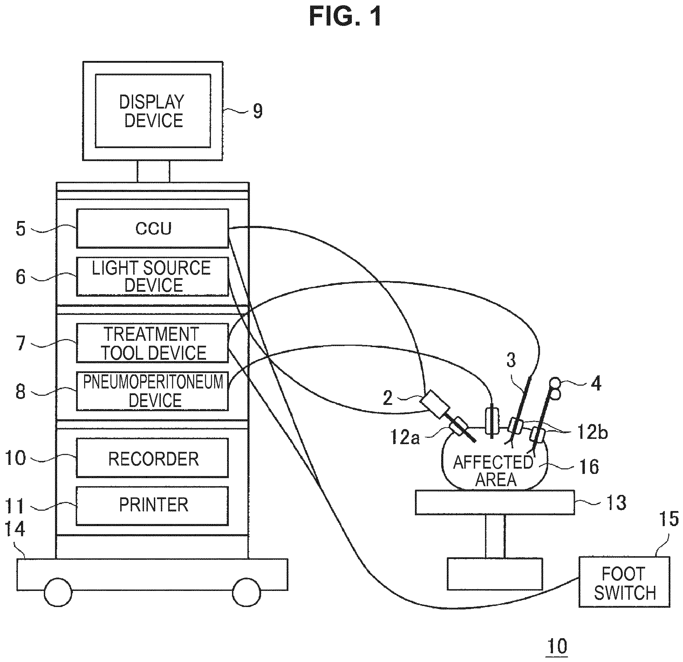

First, a schematic configuration of a system according to one embodiment of the present disclosure will be described with reference to FIG. 1. In recent years, in a field site of medical treatment, an endoscopic operation is performed in place of a traditional open abdominal operation. For example, in the case of performing operation of an abdominal part, an endoscopic operation system 10 disposed in an operating room as shown in FIG. 1 is used. Instead of opening an abdominal part by cutting an abdominal wall as in the traditional operation, hole-opening tools referred to as trocars 12a and 12b are attached to several locations on an abdominal wall, and an abdominoscope (hereinafter, also referred to as an endoscope) 2, an energy treatment tool 3, forceps 4, and the like are inserted into a body from holes provided in the trocars 12a and 12b. Then, while an image of an affected area (tumor, etc.) 16 that has been captured as a video by the endoscope 2 is viewed in real time, a treatment of cutting off the affected area 16 by the energy treatment tool 3 or the like, for example, is performed. The endoscope 2, the energy treatment tool 3, and the forceps 4 are held by an operator, an assistant, a scopist, a robot, or the like. Note that although a hard endoscope is illustrated as the endoscope 2, the endoscope 2 may be a soft endoscope. Also, the system according to the present disclosure relates to an imaging instrument, as in an endoscopic system as shown in FIG. 1, that includes an illumination device and an imaging device that acquires an image under a dark environment in which external light such as ambient light is blocked, and the system includes an observation device such as an industrial endoscope device, a medical endoscope device, or a microscopic device.

In an operating room in which such an endoscopic operation is performed, a cart 14 on which devices for the endoscopic operation and equivalents are mounted, a patient bed 13 on which a patent lies, a foot switch 15, and the like are disposed. On the cart 14, for example, devices such as a camera control unit (CCU) 5, a light source device 6, a treatment tool device 7, a pneumoperitoneum device 8, a display device 9, a recorder 10, and a printer 11 are placed as medical devices.

An image signal of the affected area 16 imaged through an observation optical system of the endoscope 2 is transmitted to the CCU 5 via a camera cable, subject to signal processing in the CCU 5, and then, output to the display device 9, on which an endoscopic image of the affected area 16 is displayed. The CCU 5 may be wirelessly connected to the endoscope 2, in addition to being connected to the endoscope 2 via the camera cable. The light source cable 6 is connected to the endoscope 2 via a light guide cable, and can irradiate, to the affected area 16, light beams with various wavelengths by switching. The treatment tool device 7 is a high frequency output device that outputs a high frequency current to the energy treatment tool 3 that cuts off the affected area 16 using electrical heat. The pneumoperitoneum device 8 includes an air supply means and an air suction means, and supplies air into, for example, an abdominal region inside the body of the patient. The foot switch 15 controls the CCU5, the treatment tool device 7, and the like using a foot manipulation performed by an operator, an assistant, or the like, as a trigger signal.

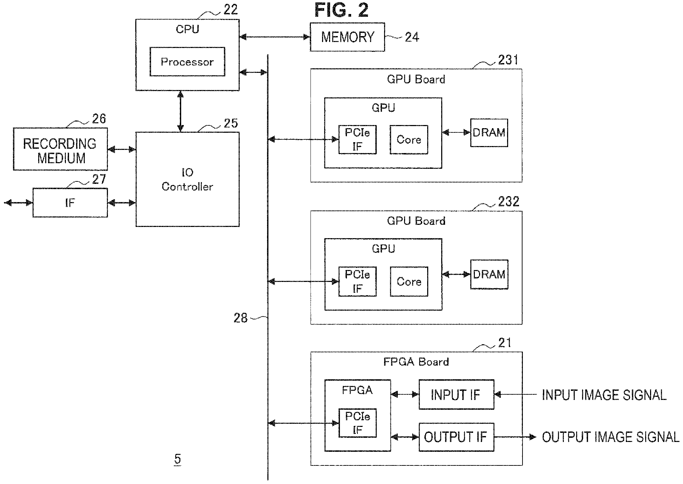

FIG. 2 is an explanatory diagram showing one example of a hardware configuration of the CCU5 in FIG. 1. The CCU5 includes, for example, a FPGA board 212, a CPU 22, GPU boards 231, 232, a memory 24, an IO controller 25, a recording medium 26, and an interface 27. In addition, the FPGA board 21, the CPU 22, and the GPU boards 231, 232 are connected via a bus 28, for example. The FPGA board 21 includes, for example, a FPGA (Field Programmable Gate Array), an input interface (input IF) in which an input image signal is input from the endoscope 2 in FIG. 1, and an output interface (output IF) that outputs an output image signal to the display device 9 in FIG. 1. In the input interface (input IF), the input image signal is input from an imaging element provided in the endoscope 2.

The CPU 22 and the GPU boards 231, 232 perform a variety of processes by executing various types of software such as associated software. The CPU 22 includes a processor. Each of the GPU boards 231, 232 includes a GPU (Graphics Processing Unit) and a DRAM (Dynamic Random Access Memory).

Various data such as data corresponding to the input image signal from the endoscope 2, the output image signal to the display device 9 and so on are stored in the memory 24. The CPU 22 serves to control writings and readings of various types of data to the memory 24.

The CPU 22 divides data stored in the memory 24, according to image data stored in the memory 24, processing capabilities of GPU boards 231, 232, and processing contents. Then, each GPU of the GPU boards 231, 232 implements a predetermined process to the data to be supplied after the division, and outputs the processed results to the CPU 22.

The IO controller 25 serves to control transmission of signals between the CPU 22, and the recording medium 26 and the interface 27.

The recording medium 26 functions as a storage unit (not shown), and stores a variety of data such as image data and various types of applications. Here, for example, a solid state drive and so on is specified as the recording medium 26. Additionally, the recording medium 26 may be detachable from the CCU 5.

As the interface 27, for example, an USB (Universal Serial Bus) terminal and a processing circuit, a LAN (Local Area Network) terminal and a transmit/receive circuit, and so on are specified.

Note that a hardware configuration of the CCU 5 is not limited to the configuration as shown in FIG. 2. For example, although an example in which the GPU boards 231, 232 are two is shown in FIG. 2, it may be the number of two or more. In addition, in a case where the CPU 22 has a function of the GPU, the CCU 5 may not include the GPU boards 231, 232. Through the use of the endoscopic operation system 10 as described above, it becomes possible to achieve an operation procedure that suppresses invasiveness that is a major demerit in a surgical operation.

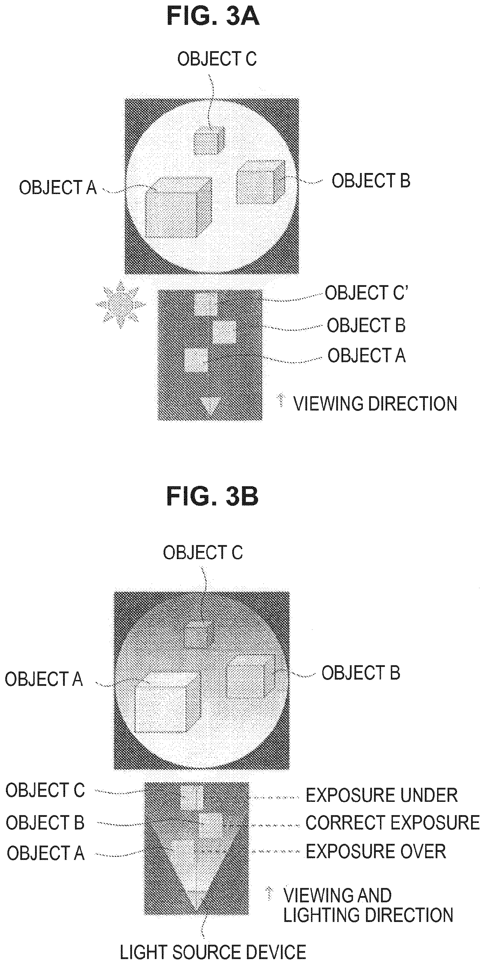

As a first problem, including the endoscopic observation as described above, that is universal in general devices that perform a dynamic observation by a single illumination device a closed space in which external light (ambient light) is blocked, it is specified to ensure a wide dynamic range (DR) of a subject. FIG. 3A is a schematic diagram showing a state in which a subject is lighted with uniform illuminance by the sun's rays such as under a natural environment. In addition, FIG. 3B is a schematic diagram showing a state in which a subject is lighted by a single light source in a blocked state of external light as in an endoscopic observation. In FIG. 3A and FIG. 3B, an upper-stage figure thereof shows a state as objects A, B, and C are viewed from a viewing direction, while a lower-stage figure thereof shows a state as the viewing direction is viewed from the top. In the state shown in FIG. 3A, as long as there are any objects having the same reflectivity to be lighted uniformly by the sun's rays, they can be seen at the same luminance irrespective of distance. On the other hand, in the state shown in FIG. 3B, since the illuminance varies greatly depending on a distance between the light source device and the subject, it becomes difficult to put the whole field of view in a correct exposure condition.

As an existing technique with respect to the first problem, as in, for example, Patent Literature 1 as mentioned previously, there is a technique that presents a video of correct exposure according to distance information. However, in this method, even when correct illuminance to a subject can be implemented, a video suitable for observation cannot be presented due to a specular reflection component from the subject.

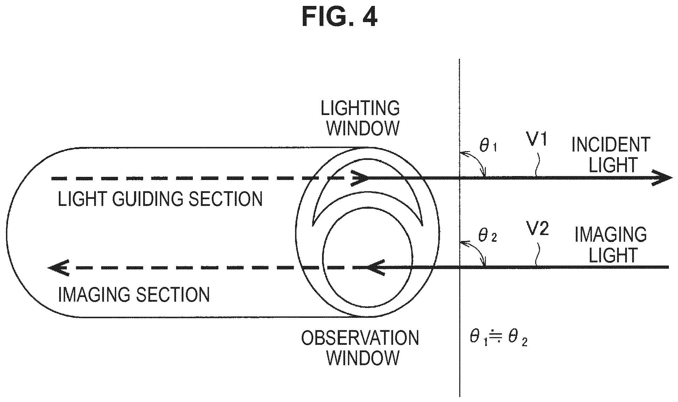

In addition, as a second problem in the observation using the system as shown in FIG. 1, there is reflection due to the specular reflection component. FIG. 4 is a schematic diagram showing a structure of an endoscopic device. For example, when the endoscope device as shown in FIG. 4 is given as a specific example, an angle (optical axis) formed between an incident light vector v1 from a light source and an imaging light vector v2 to an imaging section is liable to be in parallel, and a specular reflection easily occurs from a structural reason. On this occasion, an intensity of the specular reflection component to an intensity of a diffusion reflection component having color information of a subject is very high; when the specular reflection component is mixed in a light beam of imaging light incident on an imaging section (reflection light from the subject), a pixel value will be easily saturated, which makes it impossible to acquire information regarding the subject. Granted that the amount of exposure is adjusted to prevent saturation of the specular reflection component, the intensity of the diffusion reflection component including subject color information and so on that serves as important information in an observation is too low to acquire the diffusion reflection component. In addition, the occurrence of the specular reflection with high frequency not only hinders the observation but also gives much stress to an eye of a viewer. Therefore, when a correct observation environment is provided, the specular reflection component also has to be considered.

As an existing technique with respect to the second problem, there is a technique of cutting off the specular reflection component through the use of a polarizing plate as in Patent Literature 2, for example, described previously. However, in the technique described in Patent Literature 2, a reflection-absence image having no specular reflection component to be acquired is difficult to be used for normal observation purposes because it is different in texture and impression from a normally photographed image having the specular reflection component (reflection-presence image). In addition, in the technique described in Patent Literature 2, as a configuration for removal of the specular reflection component, since the polarizing plate is intervened for each of irradiated light and reflected light, the amount of light entering an imaging section will be less than 1/4 with respect to the amount of irradiation light; there is a problem that S/N of a video is significantly impaired.

In view of the above point, in the present embodiment, the imaging light from the subject is resolved to a first reflection light including the specular reflection component and the diffusion reflection component, and a second reflection light including only the diffusion reflection component with the specular reflection component cut off; the reflection-presence image and the reflection-absence image are acquired from the first reflection light and the second reflection light, respectively, to produce a synthesized image suitable for an observation.

In the following, a configuration example will be described such that the imaging light from the subject is resolved to the first reflection light and the second reflection light to be acquired as the reflection-presence image and the reflection-absence image, respectively.

### [2. Configuration of Endoscope in accordance with Present Embodiment]

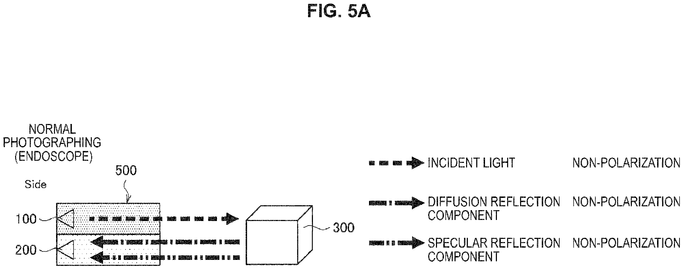

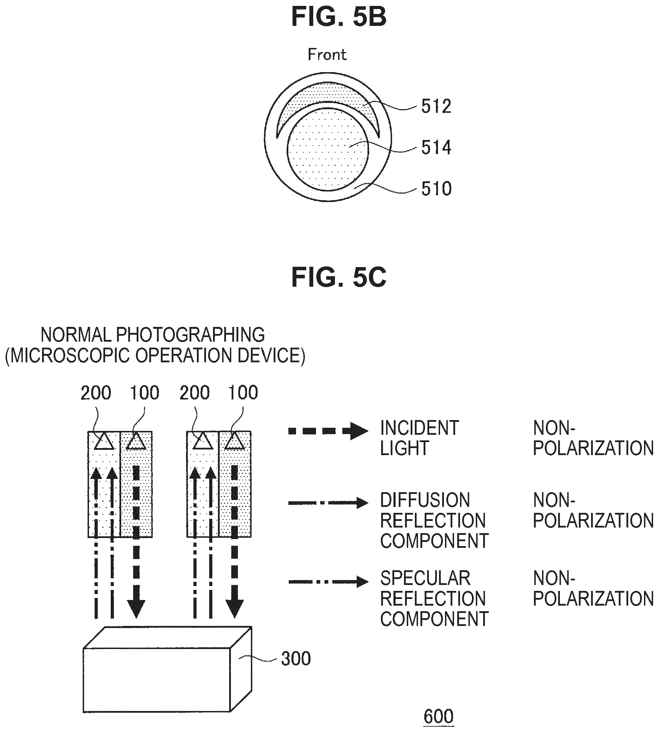

FIG. 5A to FIG. 5C each are a schematic diagram showing a relationship between irradiation light and incident light during endoscope observation in normal photographing. FIG. 5A shows a case of an endoscope device 500, and incident light irradiated from a light guiding section 100 toward a subject 300 is reflected at the subject 300, and imaging light by the reflection is imaged in an imaging section 200. The imaging section 200 includes an imaging element such as a CMOS sensor. FIG. 5B shows a state as the endoscope device 500 in FIG. 5A is seen from a subject side. As shown in FIG. 5B, in a lens barrel 510 of the endoscope device 500, an irradiation window 512 to be irradiated by the incident light and an observation window 514 on which the imaging light is incident are provided. FIG. 5C shows an example of a microscopic operation device 600, where a plurality of units each having a light guiding section 100 and the imaging section 200 are provided.

The incident light is light not polarized (non-polarization) in normal photographing as shown in FIG. 5A and FIG. 5B. The imaging light produced when the irradiated light falls on the subject 300 contains a diffusion reflection component in which polarization is resolved by diffused reflection inside an object (non-polarization, as shown by a dashed-dotted line in FIG. 5A and FIG. 5C) and a specular reflection component diffused immediately on the surface of the object (non-polarization, as shown by a dashed-two dotted line in FIG. 5A and FIG. 5C).

As a premise, in FIG. 5A to FIG. 5B, external light (ambient light) is not included in a light source that lights the subject in imaging. In addition, as shown in FIG. 4, optical axis directions of the irradiated light and the incident light are parallel to each other. The present embodiment is not limited to a configuration of the endoscope device 500; as long as any observation device that satisfies such conditions is provided, it is applicable similarly to the microscopic operation device 600 as shown in FIG. 5C and any other device configurations. In the following, a configuration of the endoscope device 500 taken as an example will be described.

### [3. Example of Spatial Division System]

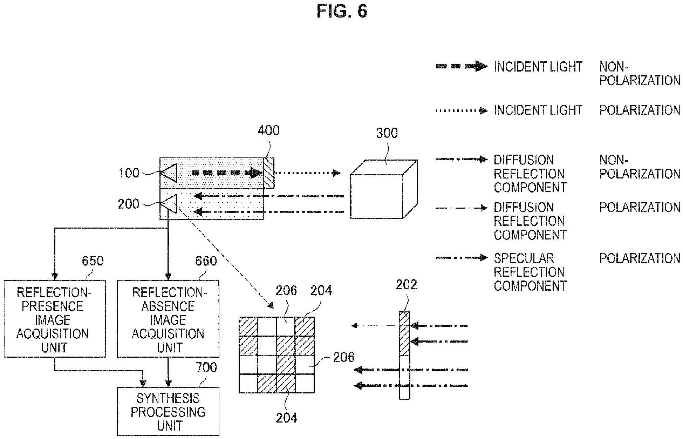

As a methodology of acquiring the first reflection light and the second reflection light, a method using the spatial division system and a method using a time division system are given. FIG. 6 is a schematic diagram showing a methodology using the spatial division system. First, incident light (non-polarization, as shown by a thick broken line in FIG. 6) that is illumination light from a first light source is irradiated from the light guiding section. The incident light is polarized at a first polarizer (polarizing plate) 400 having a first polarization angle to give incident light having the first polarization angle (polarization, as shown by a thin broken line in FIG. 6). The incident light falls on the subject 300 to produce imaging light from the subject 300. The imaging light produced on this occasion contains a diffusion reflection component in which polarization is resolved by diffused reflection inside an object (non-polarization, as shown by a thick dashed-dotted line in FIG. 6) and a specular reflection component diffused immediately on the surface of the object and constituted by polarized light keeping the first polarization angle (polarization, as shown by a thick dashed-two dotted line in FIG. 6). In this way, polarization state of diffusion reflection component having color information of an image is resolved to be diffused inside the object. On the other hand, polarization state of the specular reflection component is kept to have the first polarization angle that is the same as the incident light.

Subsequently, the imaging light arrives at the observation window 514 located at the tip of the lens barrel 510; on this occasion, in the imaging section 200 placed inside the lens barrel 510, as shown in FIG. 6, a polarization pixel 204 provided with a second polarizer 202 having a second polarization, and a normal pixel 206 not provided with the second polarizer 202 are arranged in a spatially dispersed form. The second polarizer 202 has a second polarization angle orthogonal to the first polarization angle.

In the polarization pixel 204 provided with the second polarizer 202, the diffusion reflection component (non-polarization, as shown by a thick dashed-dotted line in FIG. 6) is transmitted to give diffusion reflection component (non-polarization, as shown by a thin dashed-dotted line in FIG. 6). In addition, the specular reflection component (polarization, as shown by a thick dashed-two dotted line in FIG. 6) is cut off by the second polarizer 202. On the other hand, in the normal pixel 206 not provided with the polarizer, the diffusion reflection component (non-polarization, as shown by a thick dashed-dotted line in FIG. 6) and the specular reflection component (polarization, as shown by a thick dashed-two dotted line in FIG. 6) arrives at the normal pixel 206 without change. From the above, the specular reflection component is cut off from the light beam that arrives at the polarization pixel 204, while the specular reflection component is included in the light beam that arrives at the normal pixel 206.

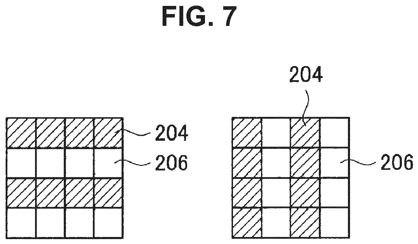

Accordingly, the image produced from only the polarization pixel 204 becomes the reflection-absence image, while the image produced from only the normal pixel 206 becomes the reflection-presence image. FIG. 7 is a schematic diagram showing a spatial arrangement of the polarization pixel 204 and the normal pixel 206. The arrangement of the polarization pixel 204 and the normal pixel 206 may be arranged in a zigzag manner, as shown in FIG. 6, or may be arranged in a horizontally or vertically divided manner, more simply as shown in FIG. 7.

As shown in FIG. 6, image information acquired in the imaging section 200 is sent to a reflection-presence image acquisition unit 650 and a reflection-absence image acquisition unit 660. In the reflection-presence image acquisition unit 650, the reflection-presence image produced from the normal pixel 206 is acquired. In addition, in the reflection-absence image acquisition unit 660, the reflection-absence image is acquired from the polarization pixel 204. The acquired reflection-presence image and reflection-absence image are synthesized in a synthesis processing unit 700. The synthesis processing unit 700 will be described later.

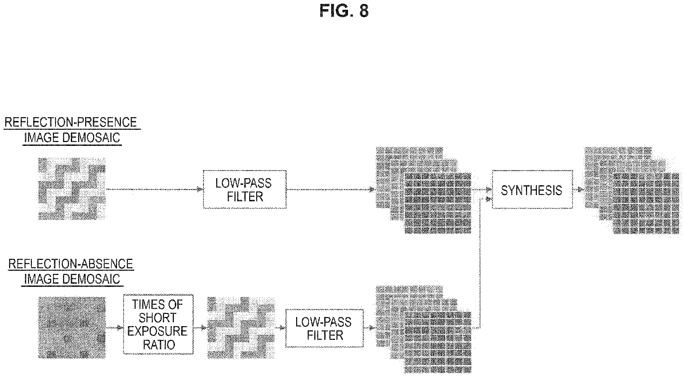

FIG. 8 is a schematic diagram showing a methodology for synthesizing the reflection-presence image and the reflection-absence image acquired by the spatial division system. In a case where the reflection-presence image and the reflection-absence image are acquired by the spatial division system, a phase (pixel) is put in short state at each signal, and it is thus necessary to interpolate the phase by means of demosaic processing. Also, since the reflection-absence image is lowered in luminance as compared to the reflection-presence image due to the passing of the second polarizer 202, it is multiplied by a gain corresponding to times of exposure ratio to be increased by such a luminance lowered portion. Then, each of the reflection-presence image and the reflection-absence image increased in luminance is passed through a low-pass filter, and then synthesized in the synthesized unit. Note that use rates of the reflection-presence image and the reflection-absence image in the synthesis will be described later. FIG. 8 shows a flow that produces an interpolation pixel by means of the low-pass filter; however, an advanced algorithm such as IGV or LMMSE may is applied thereto to produce the interpolation pixel. In addition, for the purpose of compensating resolution, from a state having information on all phases with mixture of the reflection-presence image and the reflection-absence image, a demosaic of the reflection-presence image + the reflection-absence image is combined with a demosaic-implemented result, so that a result synthesized in combination with the reflection-presence image and the reflection-absence image may be provided as an output image.

### [4. Example of Time Division System]

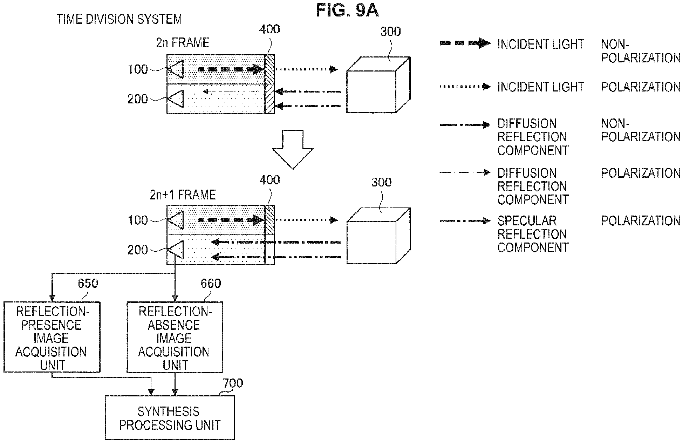

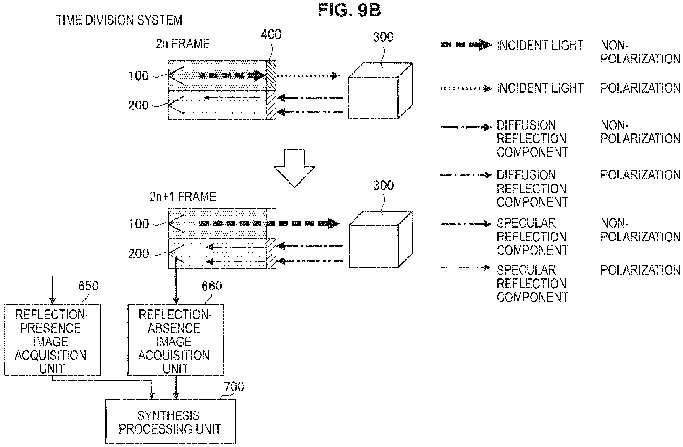

FIG. 9A and FIG. 9B each are a schematic diagram showing a methodology using a time division system. The time division system are roughly divided into two systems of a first system as shown in FIG. 9A and a second system as shown in FIG. 9B.

First, in the first system, as shown in FIG. 9A, incident light (non-polarization, as shown by a thick broken line in FIG. 9A) that is illumination light from a first light source is irradiated from a light guiding section 100. The incident light is polarized at a first polarizer 400 having a first polarization angle to give incident light having the first polarization angle (polarization, as shown by a thin broken line in FIG. 9A). The incident light falls on the subject 300 to produce imaging light from the subject 300. The imaging light produced on this occasion contains a diffusion reflection component in which polarization is resolved by diffused reflection inside an object (non-polarization, as shown by a thick dashed-dotted line in FIG. 9A) and a specular reflection component diffused immediately on the surface of the object and constituted by polarized light keeping the first polarization angle (polarization, as shown by a thick dashed-two dotted line in FIG. 9A). In the first system, the first polarizer 400 is always provided.

Subsequently, the imaging light arrives at the observation window 514 located at the tip of the lens barrel 510; in accordance with a shutter timing, the second polarizer 202 appears on the subject side of the imaging section 200 only in a 2n frame (even number frame), while no second polarizer 202 appears thereon in a 2n+1 frame (odd number frame). For this reason, since in the 2n frame, the specular reflection component of the imaging light does not pass through the second polarizer 202, the reflection-absence light is acquired. On the other hand, in the 2n+1 frame, the specular reflection component arrives at the imaging section 200, so that the reflection-presence image is acquired.

On the other hand, in the second system, as shown in FIG. 9B, the incident light that is the illumination light from the first light source arrives at the irradiation window 512; on this occasion, in accordance with the shutter timing, the first polarizer 400 appears thereon only in the 2n frame (even number frame). The second polarizer 202 is always provided. In the 2n frame, the illumination light polarized at the first polarizer 400 falls on the subject 300 to produce imaging light including the specular reflection component keeping the polarization state, and the specular reflection component is cut off at the second polarizer 202. On the other hand, since in the 2n+1 frame (odd number frame), no first polarizer 400 appears thereon, imaging light containing the specular reflection component of non-polarization is produced, and the specular reflection component of non-polarization passes through the second polarizer 202. In the 2n frame in which the specular reflection component of the imaging light does not pass through the second polarizer 202, the reflection-absence image is acquired, while in the 2n+1 frame in which the specular reflection component of the imaging light passes through the second polarizer 202, the reflection-presence image is acquired. On this occasion, instead of the appearance of the first polarizer 400 in accordance with the shutter timing, the illumination light from the first light source may be changed into that from a second light source that irradiates linearly polarized light having the same polarization angle as that of the first polarizer 400. Note that appearance and retraction of the first polarizer 400 and the second polarizer 202 can be controlled by provision of a mechanical shutter.

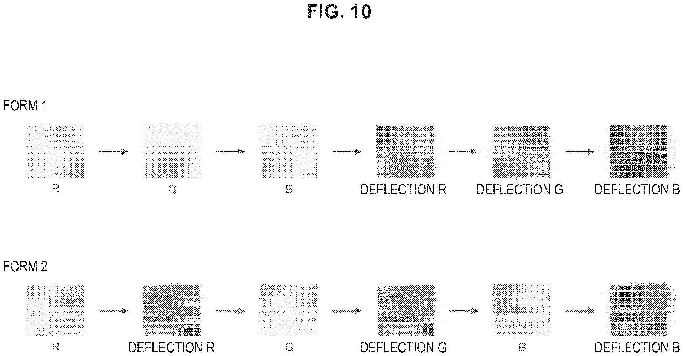

FIG. 10 is a schematic diagram showing an example in which for acquisition of the reflection-presence image and the reflection-absence image in the time division system, a switch between the first polarizer 400 and the second polarizer 202 is performed together with a switch between color filters (R, G, B). In the case of the time division system, instead of IN/OUT in a polarized filter, three reflection images and three reflection-absence images may be acquired such that the color filters (R, G, B, polarization R, polarization R, polarization B) are changed in a field sequential form. Form 1 as shown in FIG. 10 shows a field sequential form that switches 6 color filters in a manner of R -> G -> B -> polarization R -> polarization G -> polarization B. The sequence in the field sequential form of the color filters may be Form 1 as shown in FIG. 10, or may be provided as R -> polarization R -> G -> polarization G -> B -> polarization B as shown in Form 2.

### [5. Example of Light Beam Split System]

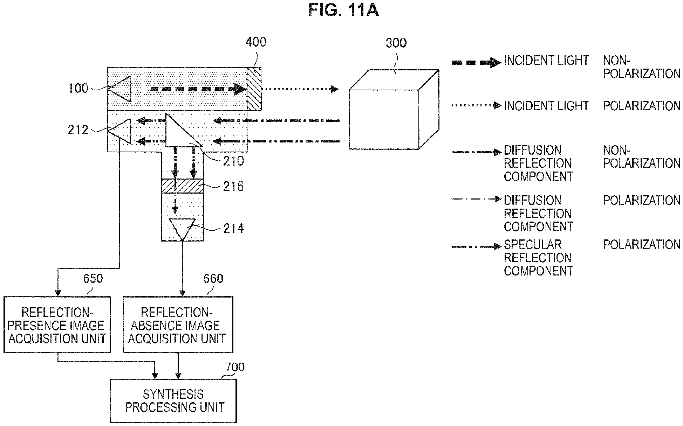

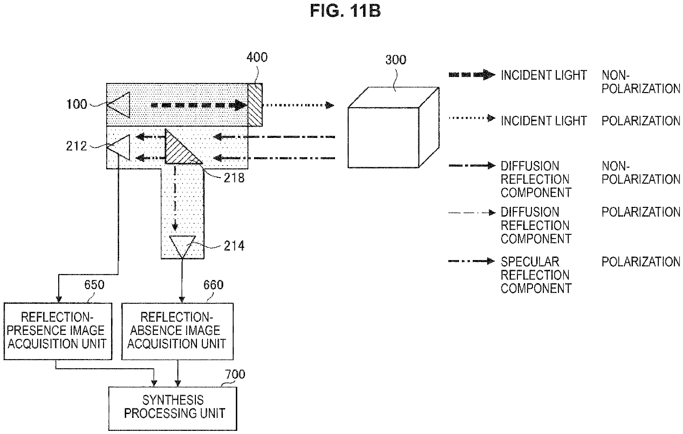

FIG. 11A and FIG. 11B each are a schematic diagram showing a methodology using a light beam split system. FIG. 11A shows a case using a common prism as a beam splitter, and FIG. 11B shows a case using a polarization beam splitter. In an example as shown in FIG. 11A, first, incident light (non-polarization, as shown by a thick broken line in FIG. 11A) from a first light source is irradiated from a light guiding section 100. The incident light is polarized at a first polarizer 400 having a first polarization angle to give incident light having a first polarization angle (polarization, as shown by a thin broken line in FIG. 11A). The incident light falls on a subject 300 to produce imaging light from the subject 300. The imaging light produced on this occasion contains a diffusion reflection component in which polarization is resolved by diffused reflection inside an object (non-polarization, as shown by a thick dashed-dotted line in FIG. 11A) and a specular reflection component diffused immediately on the surface of the object and constituted by polarized light keeping the first polarization angle (polarization, as shown by a thick dashed-two dotted line in FIG. 11A).

Subsequently, the imaging light arrives at the observation window 514 located at the tip of the lens barrel 510. The imaging light is evenly split into two light beams by a beam splitter 210 placed inside the lens barrel 510: one light beam is incident on a first imaging section 212 without change, so that the reflection-presence image is acquired; and the other light beam passes through a second polarizer 216 arranged immediately after the beam splitter 210, and thereafter is incident on a second imaging section 214. Since the second polarizer 216 has a polarization angle orthogonal to the first polarization angle, the reflection-absence image can be acquired.

Although the beam splitter 210 may be a normal prism, as shown in FIG. 11B, it may be a polarization beam splitter 218 having a horizontal or vertical polarization angle orthogonal to the first polarization angle of the first polarizer 400. In this case, the specular reflection component contained by the one light beam is parallel to the polarization angle of the polarization beam splitter 218 to be thus passed over, and arrives at one of the first imaging section 212 or the second imaging section 214, so that the reflection-presence image is acquired. In addition, the specular reflection component involved in the other light beam becomes perpendicular to the polarization angle of the polarization beam splitter 218 to be thus cut off, and arrives at the other of the first imaging section 212 or the second imaging section 214, so that the reflection-absence image is acquired. Note that although the above methodology is described as one mode in a method of acquiring the reflection-absence image and the reflection-presence image, the reflection-absence image and the reflection-presence image may be acquired by a method other than the above.

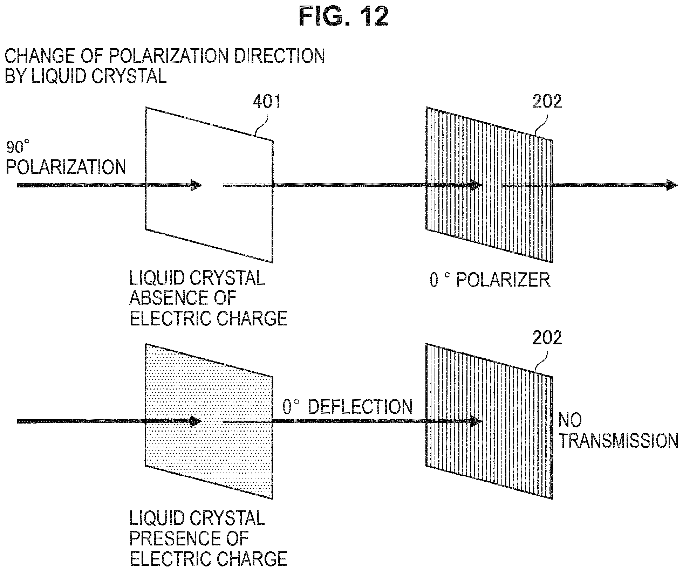

FIG. 12 is a schematic diagram showing an example, instead of preparing two polarizers (for example, 0 degrees (°) and 90 degrees), in which a direction of polarization is changed through the use of a phase difference plate 401 instead of the first polarizer 40. In a case where liquid crystal is used as the phase difference plate, no electric charge is applied to the liquid crystal in a case of acquiring the reflection-presence image. In this way, polarized light is passed over the second polarizer 202, so that the reflection-presence image can be acquired. In a case of acquiring a reflection-absence image, the electric charge is applied to the liquid crystal to shift the polarization direction by 90 degrees. In this way, the polarized light is cut off by the second polarizer 202. The amount of light in the reflection image (diffusion reflection component) is lowered according to a dimming rate of the polarizer. In principle, the example acquires the amount of light that is equivalent to that of the system using the polarization beam splitter in the light beam split system.

### [6. Synthesis of Reflection-Absence Image and Reflection-Presence Image]

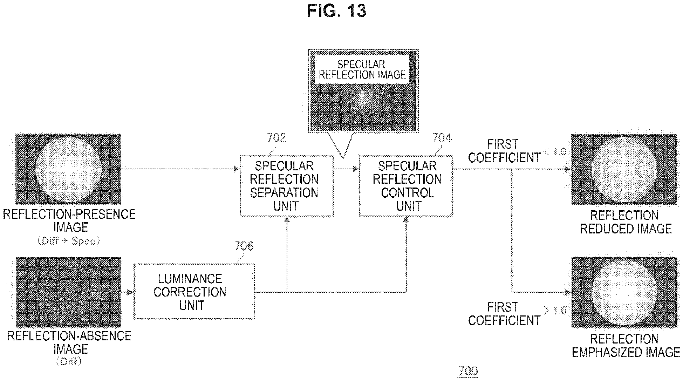

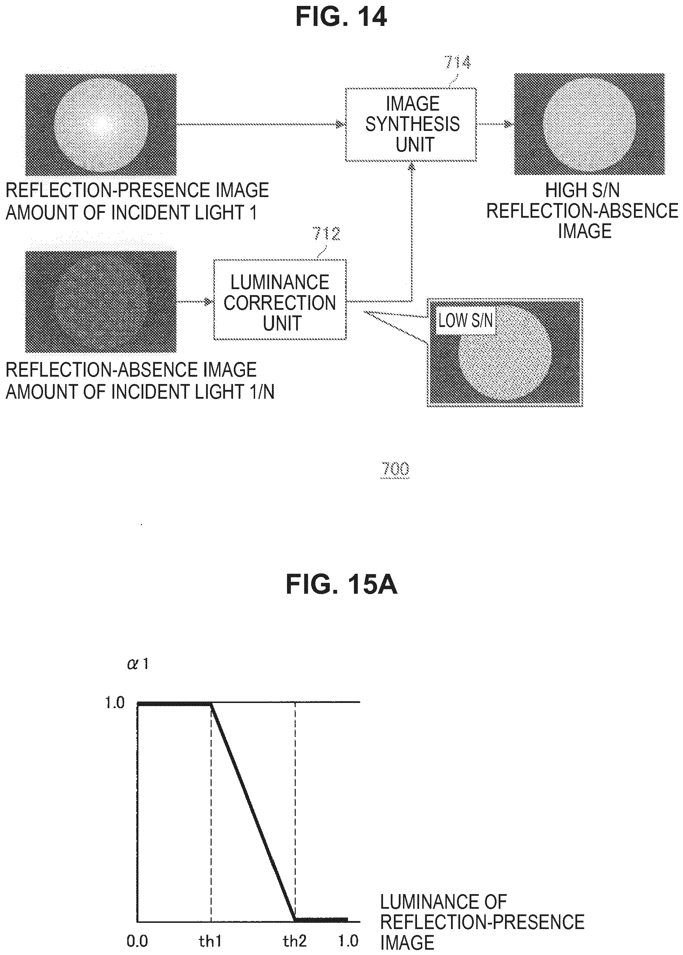

Next, on the basis of FIG. 13 and FIG. 14, a synthesis processing unit 700 that produces a synthesized image from a reflection-absence image and a reflection-presence image will be described. In FIG. 13, a method that displays as a synthesized image a specular reflection control image adaptive to an observation purpose will be described. The reflection-absence image and the reflection-presence image acquired in an imaging section 200 are acquired in a reflection-presence image acquisition unit 650 and a reflection-absence image acquisition unit 660 to be input to the synthesis processing unit 700. As shown in FIG. 13, the synthesis processing unit 700 includes a specular reflection separation unit 702, a specular reflection control unit 704 and a luminance correction unit 706.

As shown in FIG. 13, the reflection-presence image is input to the specular reflection separation unit 702, while the reflection-absence image is input to the luminance correction unit 760. First, for the purpose of setting a luminance level of the acquired reflection-absence image to that of the reflection-presence image, a broad luminance of the reflection-absence image is adjusted by the luminance correction unit 706. Here, for example, from a dimming rate when a light beam of non-polarization is transmitted through a polarizer, the luminance level of the reflection-absence image is set to that of the reflection-presence image. The luminance to be adjusted on this occasion may be performed uniformly on a screen, or determined for each area or pixel to be applied thereto. As a method of making the determination for each area or pixel, it is preferable to use, for example, a ratio in pixels of note (phase) or area of note between the reflection-presence image and the reflection-absence image. On this occasion, a case where the pixel or area of the reflection-presence image contains a specular reflection component is eliminated because a pixel value becomes extremely higher, and it is intended that the pixel or area in calculation of the ratio avoids containing the specular reflection component. The reflection-absence image adjusted in luminance is input to the specular reflection separation unit 702 and the specular reflection control unit 704.

Subsequently, for the purpose of extracting the specular reflection component, the specular reflection separation unit 702 calculates a difference between the reflection-presence image and the reflection-absence image adjusted in luminance to extract the resultant as a specular reflection image. The extracted specular reflection image is input to the specular reflection control unit 704.

Next, for the purpose of producing an image according to an observation purpose, the specular reflection control unit 704 multiplies the extracted specular reflection image by a first coefficient A, and add the resultant to the reflection-absence image adjusted in luminance level to thus output a synthesized image. On this occasion, when the first coefficient A to be multiplied with the specular reflection image is 0.0 or more and less than 1.0, an image with specular reflection reduced or lost is given, which can suppress saturation of image information and improve visibility. On the other hand, when the first coefficient A becomes 1.0 or more, the specular reflection is emphasized, so that an image strong in texture and stereoscopic effect can be obtained.

Next, on the basis of FIG. 14, a method for providing a synthesized image having a higher S/N of dark section (low luminance area) will be described such that the reflection-absence image and the reflection-presence image are treated as a short exposure image and a long exposure image, respectively, to implement a HDR synthesis. A synthesized image producing unit 700 as shown in FIG. 14 includes a luminance correction unit 712 and an image synthesis unit 714. As shown in FIG. 14, the reflection-presence image is input to the image synthesis unit 714, while the reflection-absence image is input to the luminance correction unit 712. First, for the purpose of setting an exposure amount in principle to the reflection-absence image, the luminance level of the reflection-absence image is adjusted by the luminance correction unit 712. On this occasion, a second coefficient to be added thereto as a gain in the luminance to be adjusted is given as a unique value uniformly in screen. The second coefficient is found, for example, from a dimming rate when a light beam of non-polarization is transmitted through a polarizer.

Next, the reflection-absence image adjusted in luminance and the reflection-presence image are synthesized. In the synthesis, with respect to a part without specular reflection, the reflection-presence image has a higher S/N ratio, and thus the reflection-presence image is used. On the other hand, with respect to a part in which a subject is not seen due to reflection, the reflection-absence image has more image information even when having a lower S/N ratio, and thus the reflection-absence image is used. When the synthesis is implemented from such viewpoints, the reflection-absence image having a higher S/N ratio can be obtained.

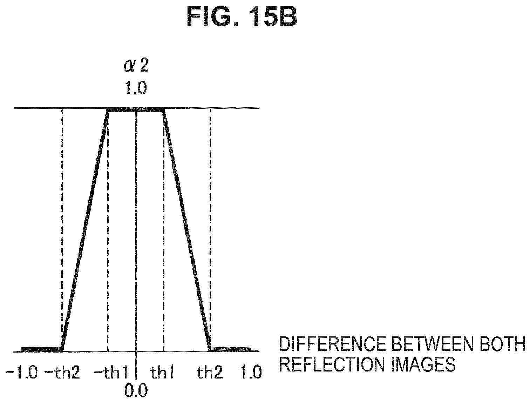

FIG. 15A and FIG. 15B are characteristic graphs showing use rate coefficients α1 and α2 in the synthesis. From the above viewpoints, as shown in FIG. 15A, the use rate α1 of the reflection-presence image or the reflection-absence image can be determined, for example, by a luminance of the reflection-presence image. In a part where the luminance of the reflection-presence image is high, the specular reflection is large; thus, the use rate coefficient α1 of the reflection-presence image is made lower, and the use rate of the reflection-absence image is made higher. On the other hand, in a part where the luminance of the reflection-presence image is low, the specular reflection is small; thus, the use rate coefficient α1 of the reflection-presence image is made higher, and the use rate of the reflection-absence image is made lower. Such a synthesis can be performed for each area or each pixel of an image.

On the other hand, different from a synthesis of images in normal long-short-exposure photographing, reflection components are different to be contained in both reflection images of the reflection-absence image and the reflection-presence image; thus, the luminance adjusted by the luminance correction unit 706 does not always coincide with that of the reflection-presence image. For this reason, in some cases, the synthesis of both reflection images cannot be achieved only by a concept as shown in FIG. 15A.

Accordingly, as shown in FIG. 15B, as a coefficient for finding a synthesis ratio, a coefficient α2 that uses as a weight a difference between both reflection images can be used. In a case where a difference between the reflection-absence image adjusted in luminance and the reflection-presence image is large, it can be considered that the difference is caused due to the specular refection component. Since it is considered that the difference between both reflection images is a distinction derived from the specular refection component, the synthesis ratio is weighted by the coefficient α2 such that the larger the difference, the higher the use rate of the image having a reduced specular reflection component, that is, the reflection-absence image. When the coefficient α2 is calculated, a ratio may be used, instead the difference. In this regard, in the case, it is preferable that a scheme is made to prevent the use rate of the reflection-absence image in the dark part from increasing.

In the present embodiment, different from the synthesis of images in the normal long-short-exposure photographing (HDR synthesis), the synthesis of reflection-presence image and the reflection-absence image can adjust the specular reflection component at optimum. In this way, in particular, in a case where the specular reflection component is large, it can be suppressed that the pixel value is completely saturated. On the other hand, in the synthesis of images in the normal long-short-exposure photographing, it becomes impossible to obtain the image information when the pixel value is completely saturated due to the specular reflection component, and even when the pixel value is not saturated, it becomes difficult to obtain sufficient image information. Therefore, according to the present embodiment, the S/N in the image can be made higher as compared to the synthesis in the normal long-short-exposure photographing; it becomes possible to surely suppress degradation in image quality due to the specular reflection component.

As described above, according to the present embodiment, an image suitable for an observation can be presented such that the reflection-presence image and the reflection-absence image are acquired to synthesize both reflection images, and the intensity of the specular reflection component of the subject is adjusted according to a purpose. In this way, even when the specular reflection component is strong, it becomes possible to surely suppress degradation in image quality by suppression of the specular reflection component. In addition, it becomes possible to enhance texture of the image such that the use rate of the specular reflection component is increased according to a purpose. Moreover, an image with a high S/N and high visibility in the dark part can be presented by the synthesis of both reflection images.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) An endoscope device including:
   a reflection-presence image acquisition unit that acquires a reflection-presence image containing a specular reflection component from a subject;
   a reflection-absence image acquisition unit that acquires a reflection-absence image not containing the specular reflection component from the subject; and
   a synthesis processing unit that performs synthesis of the reflection-presence image and the reflection-absence image.
(2) The endoscope device according to (1),
   in which the synthesis processing unit synthesizes the reflection-presence image and the reflection-absence image by adding a difference between the reflection-presence image and the reflection-absence image to the reflection-absence image.
(3) The endoscope device according to (1),
   in which on the basis of luminance of the reflection-presence image, the synthesis processing unit performs the synthesis such that a use rate of the reflection-presence image is made lower as the luminance of the reflection-presence image is higher.
(4) The endoscope device according to (1),
   in which on the basis of a difference between luminance of the reflection-presence image and luminance of the reflection-absence image, the synthesis processing unit performs the synthesis such that a use rate of the reflection-presence image is made lower as the difference is larger.
(5) The endoscope device according to any one of (1) to (4),
   in which the synthesis processing unit includes a luminance correction unit that sets luminance of the reflection-absence image to luminance of the reflection-presence image.
(6) The endoscope device according to any one of (1) to (5),
   in which the synthesis processing unit performs the synthesis for each area or each pixel of an image.
(7) The endoscope device according to any one of (1) to (6), including:
   a first polarizer that changes light emitted from a light source unit to linearly polarized light, and causes the linearly polarized light to be incident on the subject; and
   a second polarizer that transmits a light beam reflected from the subject, and has a different polarization angle from the first polarizer.
(8) The endoscope device according to (7),
   in which the second polarizer has a polarization angle orthogonal to a polarization angle of the first polarizer.
(9) The endoscope device according to (7) or (8),
   in which the reflection-presence image acquisition unit acquires the reflection-presence image at a time divided, first predetermined frame,
   the reflection-absence image acquisition unit acquires the reflection-absence image at a time divided, second predetermined frame,
   one of the first polarizer and the second polarizer appears at the first predetermined frame, and
   both of the first polarizer and the second polarizer appear at the second predetermined frame.
(10) The endoscope device according to (7) or (8),
   in which the second polarizer is arranged in a polarization pixel of a plurality of pixels,
   the reflection-presence image acquisition unit acquires the reflection-presence image from a normal pixel in which the second polarizer is not arranged, and
   the reflection-absence image acquisition unit acquires the reflection-absence image from the polarization pixel in which the second polarizer is arranged.
(11) The endoscope device according to (7), including
   a beam splitter that splits the light beam reflected from the subject into two light beams,
   in which the second polarizer transmits one of the two split light beams,
   the reflection-presence image acquisition unit acquires the reflection-presence image from a light beam that has not passed through the second polarizer of the two split light beams, and
   the reflection-presence image acquisition unit acquires the reflection-absence image from a light beam that has passed through the second polarizer of the two split light beams.
(12) The endoscope device according to any one of (1) to (5), including
   a first polarizer that changes light emitted from a light source unit to linearly polarized light, and causes the linearly polarized light to be incident on the subject; and
   a polarization beam splitter that splits a light beam reflected from the subject into two light beams,
   in which the polarization beam splitter changes a polarization state in one of the two split light beams,
   the reflection-presence image acquisition unit acquires the reflection-presence image from a light beam of which the polarization state has not been changed by the polarization beam splitter of the two split light beams, and
   the reflection-presence image acquisition unit acquires the reflection-absence image from a light beam of which the polarization state has been changed by the polarization beam splitter of the two split light beams.
(13) The endoscope device according to any one of (1) to (12),
   in which an optical axis of a light beam incident on the subject is parallel to an optical axis of a light beam reflected from the subject.
(14) An image synthesis method for an endoscope device, including:
   acquiring a reflection-presence image containing a specular reflection component from a subject;
   acquiring a reflection-absence image not containing the specular reflection component from the subject; and
   performing synthesis of the reflection-presence image and the reflection-absence image.

### Reference Signs List

- 202: second polarizer
- 204: polarization pixel
- 206: normal pixel
- 210: beam splitter
- 218: polarization beam splitter
- 400: first polarizer
- 650: reflection-presence image acquisition unit
- 660: reflection-absence image acquisition unit
- 700: synthesis processing unit
- 706, 712: luminance correction unit

## Claims

1. An endoscope device comprising:
a reflection-presence image acquisition unit that acquires a reflection-presence image containing a specular reflection component from a subject;
a reflection-absence image acquisition unit that acquires a reflection-absence image not containing the specular reflection component from the subject; and
a synthesis processing unit that performs synthesis of the reflection-presence image and the reflection-absence image.

2. The endoscope device according to claim 1,
wherein the synthesis processing unit synthesizes the reflection-presence image and the reflection-absence image by adding a difference between the reflection-presence image and the reflection-absence image to the reflection-absence image.

3. The endoscope device according to claim 1,
wherein on a basis of luminance of the reflection-presence image, the synthesis processing unit performs the synthesis such that a use rate of the reflection-presence image is made lower as the luminance of the reflection-presence image is higher.

4. The endoscope device according to claim 1,
wherein on a basis of a difference between luminance of the reflection-presence image and luminance of the reflection-absence image, the synthesis processing unit performs the synthesis such that a use rate of the reflection-presence image is made lower as the difference is larger.

5. The endoscope device according to claim 1,
wherein the synthesis processing unit includes a luminance correction unit that sets luminance of the reflection-absence image to luminance of the reflection-presence image.

6. The endoscope device according to claim 1,
wherein the synthesis processing unit performs the synthesis for each area or each pixel of an image.

7. The endoscope device according to claim 1, comprising:
a first polarizer that changes light emitted from a light source unit to linearly polarized light, and causes the linearly polarized light to be incident on the subject; and
a second polarizer that transmits a light beam reflected from the subject, and has a different polarization angle from the first polarizer.

8. The endoscope device according to claim 7,
wherein the second polarizer has a polarization angle orthogonal to a polarization angle of the first polarizer.

9. The endoscope device according to claim 7,
wherein the reflection-presence image acquisition unit acquires the reflection-presence image at a time divided, first predetermined frame,
the reflection-absence image acquisition unit acquires the reflection-absence image at a time divided, second predetermined frame,
one of the first polarizer and the second polarizer appears at the first predetermined frame, and
both of the first polarizer and the second polarizer appear at the second predetermined frame.

10. The endoscope device according to claim 7,
wherein the second polarizer is arranged in a polarization pixel of a plurality of pixels,
the reflection-presence image acquisition unit acquires the reflection-presence image from a normal pixel in which the second polarizer is not arranged, and
the reflection-absence image acquisition unit acquires the reflection-absence image from the polarization pixel in which the second polarizer is arranged.

11. The endoscope device according to claim 7, comprising
a beam splitter that splits the light beam reflected from the subject into two light beams,
wherein the second polarizer transmits one of the two split light beams,
the reflection-presence image acquisition unit acquires the reflection-presence image from a light beam that has not passed through the second polarizer of the two split light beams, and
the reflection-presence image acquisition unit acquires the reflection-absence image from a light beam that has passed through the second polarizer of the two split light beams.

12. The endoscope device according to claim 1, comprising
a first polarizer that changes light emitted from a light source unit to linearly polarized light, and causes the linearly polarized light to be incident on the subject; and
a polarization beam splitter that splits a light beam reflected from the subject into two light beams,
wherein the polarization beam splitter changes a polarization state in one of the two split light beams,
the reflection-presence image acquisition unit acquires the reflection-presence image from a light beam of which the polarization state has not been changed by the polarization beam splitter of the two split light beams, and
the reflection-presence image acquisition unit acquires the reflection-absence image from a light beam of which the polarization state has been changed by the polarization beam splitter of the two split light beams.

13. The endoscope device according to claim 1,
wherein an optical axis of a light beam incident on the subject is parallel to an optical axis of a light beam reflected from the subject.

14. An image synthesis method for an endoscope device, comprising:
acquiring a reflection-presence image containing a specular reflection component from a subject;
acquiring a reflection-absence image not containing the specular reflection component from the subject; and
performing synthesis of the reflection-presence image and the reflection-absence image.
